# EUROPEAN PATENT SPECIFICATION

(11) **EP 2 833 884 B1**
(45) Date of publication and mention of the grant of the patent: **20.05.2020**
(21) Application number: 13713199.1
(22) Date of filing: 02.04.2013
(51) Int. Cl.: A61K 31/4545, A61P 27/02, A61P 11/00, A61P 37/00

(54) **USE OF CCR3-INHIBITORS**
VERWENDUNG VON CCR3-HEMMERN
UTILISATION D'INHIBITEURS DE CCR3

(30) Priority: 03.04.2012 EP 12162937
(43) Date of publication of application: 11.02.2015
(73) Proprietor: Alkahest, Inc., San Carlos, CA 94070 (US)
(72) Inventor: NIVENS, Michael Chadham, 55216 Ingelheim Am Rhein (DE); BOUYSSOU, Thierry, 55216 Ingelheim Am Rhein (DE); GOEGGEL, Rolf, 55216 Ingelheim Am Rhein (DE); SEITHER, Peter, 55216 Ingelheim Am Rhein (DE)
(74) Representative: Grund, Martin
(86) International application number: PCT/EP2013/056864
(87) International publication number: WO 2013/149986

(56) References cited:
- WO-A1-2010/115836
- WO-A1-2012/045803
- WO-A2-2006/083390
- US-A1- 2009 123 375
- ATSUNOBU TAKEDA ET AL: "CCR3 is a target for age-related macular degeneration diagnosis and therapy", NATURE: INTERNATIONAL WEEKLY JOURNAL OF SCIENCE, NATURE PUBLISHING GROUP, UNITED KINGDOM , vol. 460, no. 7252 9 July 2009 (2009-07-09), pages 225-230, XP002693828, ISSN: 0028-0836, DOI: 10.1038/NATURE08151 Retrieved from the Internet: URL:http://www.nature.com/nature/journal/v 460/n7252/pdf/nature08151.pdf [retrieved on 2009-06-14]
- BACHERT CLAUS ET AL: "Pharmacological management of nasal polyposis", DRUGS, vol. 65, no. 11, 2005, pages 1537-1552, XP002697885, ISSN: 0012-6667
- BLANCHARD C ET AL: "Eotaxin-3 and a Uniquely Conserved Gene-Expression profile in Eosinophilic Esophagitis", JOURNAL OF CLINICAL INVESTIGATION, AMERICAN SOCIETY FOR CLINICAL INVESTIGATION, US, vol. 116, no. 2, 1 February 2006 (2006-02-01), pages 536-547, XP003003549, ISSN: 0021-9738, DOI: 10.1172/JCI26679

## Description

The present invention relates to CCR3-inhibitors of formula **1,** 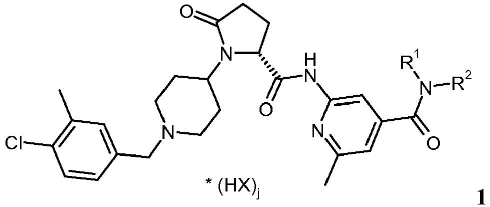 wherein
- R¹: is H, C₁₋₆-alkyl, C₀₋₄-alkyl-C₃₋₆-cycloalkyl, C₁₋₆-haloalkyl;
- R²: is H, C₁₋₆-alkyl;
- X: is an anion selected from the group consisting of chloride or ½ dibenzoyltartrate
- j: is 1 or 2.
for use as a medicament for the treatment of diseases selected from nasal polyposis, eosinophilic esophagitis, eosinophillic gastroenteritis (e.g. eosinophilic gastritis and eosinophilic ententeritis), hypereosinophilic syndrome and Churg Strauss syndrome.

### BACKGROUND INFORMATION

Chemokines are chemotactic cytokines, of molecular weight 6-15 kDa, that are released by a wide variety of cells to attract and activate, among other cell types, macrophages, T and B lymphocytes, eosinophils, basophils and neutrophils (reviewed in Luster, New Eng. J Med., 338, 436-445 (1998); Rollins, Blood, 90, 909-928 (1997); Lloyd, Curr. Opin. Pharmacol., 3, 443-448 (2003); Murray, Current Drug Targets., 7, 579-588 (2006); Smit, Eur J Pharmacol., 533,277-88 (2006)

There are two major classes of chemokines, CXC and CC, depending on whether the first two cysteines in the amino acid sequence are separated by a single amino acid (CXC) or are adjacent (CC). The CXC chemokines, such as interleukin-8 (IL-8), neutrophil-activating protein-2 (NAP2) and melanoma growth stimulatory activity protein (MGSA) are chemotactic primarily for neutrophils and T lymphocytes, whereas the CC chemokines, such as RANTES, MIP-la, MIP-1, the monocyte chemotactic proteins (MCP-1, MCP-2, MCP-3, MCP-4, and MCP-5) and the eotaxins (-1,-2, and-3) are chemotactic for, among other cell types, macrophages, T lymphocytes, eosinophils, mast cells, dendritic cells, and basophils. Also in existence are the chemokines lymphotactin-1, lymphotactin-2 (both C chemokines), and fractalkine (a CXXXC chemokine) that do not fall into either of the major chemokine subfamilies.

The chemokines bind to specific cell-surface receptors belonging to the family of G-protein-coupled seventransmembrane-domain proteins (reviewed in Horuk, Trends Pharm. Sci., 15, 159-165 (1994); Murphy, Pharmacol Rev., 54 (2):227-229 (2002); Allen, Annu. Rev. Immunol., 25, 787-820 (2007)) which are termed "chemokine receptors." On binding their cognate ligands, chemokine receptors transduce an intracellular signal through the associated trimeric G proteins, resulting in, among other responses, a rapid increase in intracellular calcium concentration, activation of G-proteins, changes in cell shape, increased expression of cellular adhesion molecules, degranulation, promotion of cell migration, survival and proliferation. There are at least eleven human chemokine receptors that bind or respond to CC chemokines with the following characteristic patterns: CCR-1 (or"CKR-1"or"CC-CKR-1") [MIP-la, MCP-3, MCP-4, RANTES] (Ben-Barruch, et al., Cell, 72, 415-425 (1993), Luster, New Eng. J. Med., 338, 436-445 (1998)); CCR-2A and CCR-2B (or "CKR-2A"/"CKR-2B"or"CC-CKR-2A"/"CC-CKR-2B") [MCP-1, MCP2, MCP-3, MCP-4, MCP-5] (Charo et al., Proc. Natl. Acad. Sci. USA, 91, 2752-2756 (1994), Luster, New Eng. J. Med., 338, 436-445 (1998)); CCR3 (or"CKR-3"or"CC-CKR-3") [eotaxin-1, eotaxin-2, RANTES, MCP-3, MCP-4] (Combadiere, et al., J. Biol. Chem., 270, 16491-16494 (1995), Luster, New Eng. J. Med., 338, 436-445 (1998)); CCR-4 (or"CKR-4" or"CC-CKR-4") [TARC, MIP-la, RANTES, MCP-1] (Power et al., J. Biol. Chem., 270, 19495-19500 (1995), Luster, New Eng. J. Med., 338, 436-445 (1998)); CCR-5 (or"CKR-5"OR"CCCKR-5") [MIP-la, RANTES, MIP-lp] (Sanson, et al., Biochemistry, 35, 3362-3367 (1996)); CCR-6 (or"CKR-6"or "CC-CKR-6") [LARC] (Baba et al., J. Biol. Chem., 272, 14893-14898 (1997)); CCR-7 (or"CKR-7"or"CC-CKR-7") [ELC] (Yoshie et al., J. Leukoc. Biol. 62, 634-644 (1997)); CCR-8 (or"CKR-8"or"CC-CKR-8") [1-309, TARC, MIP-1p] (Napolitano et al., J. Immunol., 157, 2759-2763 (1996), Bernardini et al., Eur. J. Immunol., 28, 582-588 (1998)); CCR-10 (or"CKR-10"or"CC-CKR-10") [MCP-1, MCP-3] (Bonini et al, DNA and Cell Biol., 16, 1249-1256 (1997)) ; and CCR31 (or "CKR-11" or "CC-CKR-11") [MCP-1, MCP-2, MCP-4](Schweickart et al., J Biol Chem, 275 9550-9556 (2000)).

In addition to the mammalian chemokine receptors, the Decoy receptors CCX-CKR, D6 and DARC/Duffy as well proteins expressed by mammalian cytomegaloviruses, herpes viruses and poxviruses, exhibit binding properties of chemokine receptors (reviewed by Wells and Schwartz, Curr. Opin. Biotech., 8, 741-748 (1997); Comerford, Bioessays., 29(3):237-47 (2007)). Human CC chemokines, such as RANTES and MCP-3, can cause rapid mobilization of calcium via these virally encoded receptors. Receptor expression may be permissive for infection by allowing for the subversion of normal immune system surveillance and response to infection. Additionally, human chemokine receptors, such as CXCR-4, CCR2, CCR3, CCR5 and CCR8, can act as co receptors for the infection of mammalian cells by microbes as with, for example, the human immunodeficiency viruses (HIV).

Chemokine receptors have been implicated as being important mediators of inflammatory, infectious, and immunoregulatory disorders and diseases, including asthma and allergic diseases, as well as autoimmune pathologies such as rheumatoid arthritis, Grave's disease, chronic obstructive pulmonary disease, and atherosclerosis. For example, the chemokine receptor CCR3 is expressed among others on eosinophils, basophils, TH2 cells, alveolar macrophages, mast cells, epithelial cells, microglia cells, astrocytes and fibroblasts. CCR3 plays a pivotal role in attracting eosinophils to sites of allergic inflammation and in subsequently activating these cells. The chemokine ligands for CCR3 induce a rapid increase in intracellular calcium concentration, increased GTP exchange of G-proteins, increased ERK phosphorylation, enhanced receptor internalization, eosinophil shape change, increased expression of cellular adhesion molecules, cellular degranulation, and the promotion of migration. Accordingly, agents that inhibit chemokine receptors would be useful in such disorders and diseases. In addition, agents that inhibit chemokine receptors would also be useful in infectious diseases such as by blocking infection of CCR3 expressing cells by HIV or in preventing the manipulation of immune cellular responses by viruses such as cytomegaloviruses.

Therefore, CCR3 is an important target and antagonism of CCR3 is likely to be effective in the treatment of inflammatory, eosinophilic, immunoregulatory and infectious disorders and diseases (Wegmann, Am J Respir Cell Mol Biol., 36(1):61-67 (2007); Fryer J Clin Invest., 116(1):228-236 (2006); De Lucca, Curr Opin Drug Discov Devel., 9(4):516-524 (2006)

It has been found and disclosed in WO 2010 115836 that the substituted piperidines of formula 1 are highly suitable as CCR3 antagonists, having less side effects, e.g. inhibition of norepinephrine (NET), dopamine (DAT) or serotonin reuptake transporters (5-HTT) as described by Watson PS, Bioorg Med Chem Lett., 16(21):5695-5699 (2006), or inhibition of 5HT2A, 5HT2C or Dopamine D2 receptors as described by De Lucca, J Med Chem., 48(6):2194-2211(2005), or inhibition of the hERG channel as described by De Lucca, Curr Opin Drug Discov Devel., 9(4):516-524 (2006), or inhibition of the alphalB adrenergic receptor.

Surprisingly it has now been found, that compounds of formula **1** are useful for the treatment of diseases selected from nasal polyposis and eosinophilic esophagitis.

Similarly compounds of formula **1** are useful for treating other diseases selected from eosinophillic gastroenteritis (e.g. eosinophilic gastritis and eosinophilic ententeritis), hypereosinophilic syndrome, and Churg Strauss syndrome.

### DESCRIPTION OF THE INVENTION

Object of the present invention are compounds of formula **1** 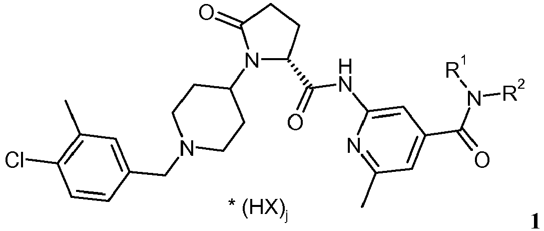 wherein
- R¹: is H, C₁₋₆-alkyl, C₀₋₄-alkyl-C₃₋₆-cycloalkyl, C₁₋₆-haloalkyl;
- R²: is H, C₁₋₆-alkyl;
- X: is an anion selected from the group consisting of chloride or ½ dibenzoyltartrate
- j: is 1 or 2;
for use as a medicament for the treatment of diseases selected from nasal polyposis, eosinophilic esophagitis, eosinophillic gastroenteritis (e.g. eosinophilic gastritis and eosinophilic ententeritis), hypereosinophilic syndrome and Churg Strauss syndrome.

Preferred are compounds of formula **1** wherein
- R¹: is H, C₁₋₆-alkyl;
- R²: is H, C₁₋₆-alkyl;
- X: is an anion selected from the group consisting of chloride or ½ dibenzoyltartrate
- j: is 1 or 2.

Preferred are compounds of formula **1** wherein
- R¹: is H, Methyl, Ethyl, Propyl, Butyl;
- R²: is H, Methyl, Ethyl, Propyl, Butyl;
- X: is an anion selected from the group consisting of chloride or ½ dibenzoyltartrate, preferably chloride;
- j: is 1 or 2, preferably 2.

Preferred are compounds of formula **1** wherein
- R¹: is H, Methyl, Ethyl, Propyl, Butyl;
- R²: is H, Methyl;
- X: is an anion selected from the group consisting of chloride or ½ dibenzoyltartrate, preferably chloride;
- j: is 1 or 2, preferably 2.

Preferred are compounds of formula **1** wherein
- R¹: is H, Methyl;
- R²: is H, Methyl;
- X: is an anion selected from the group consisting of chloride or ½ dibenzoyltartrate, preferably chloride;
- j: is 1 or 2, preferably 2.

Furthermore preferred are compounds according to the examples 1, 2, 3, 4, 5, 6, 7, 8, 9 or 10 from the table below as a di-hydrochloride. Thus, preferably X is chloride and preferably j is 2. Preferred are compounds of formula **1** for use as a medicament for the treatment of diseases selected from nasal polyposis and eosinophilic esophagitis.

Another aspect of the invention is the use of compounds of formula **1** for the treatment of diseases selected from nasal polyposis, eosinophilic esophagitis, eosinophillic gastroenteritis (e.g. eosinophilic gastritis and eosinophilic ententeritis), hypereosinophilic syndrome and Churg Strauss syndrome, preferably nasal polyposis and eosinophilic esophagitis.

Another aspect of the invention is the use of compounds of formula **1** for the manufacturing of a medicament for the treatment of diseases selected from nasal polyposis, eosinophilic esophagitis, eosinophillic gastroenteritis (e.g. eosinophilic gastritis and eosinophilic ententeritis), hypereosinophilic syndrome and Churg Strauss syndrome, preferably nasal polyposis and eosinophilic esophagitis.

### USED TERMS AND DEFINITIONS

Terms not specifically defined herein should be given the meanings that would be given to them by one of skill in the art in light of the disclosure and the context. As used in the specification, however, unless specified to the contrary, the following terms have the meaning indicated and the following conventions are adhered to.

In the groups, radicals, or moieties defined below, the number of carbon atoms is often specified preceding the group, for example, C₁₋₆-alkyl means an alkyl group or radical having 1 to 6 carbon atoms. In general, for groups comprising two or more subgroups, the first named subgroup is the radical attachment point, for example, the substituent "C₁₋₃-alkyl-aryl" means an aryl group which is bound to a C₁₋₃-alkyl-group, the latter of which is bound to the core or to the group to which the substituent is attached.

In case a compound of the present invention is depicted in form of a chemical name and as a formula in case of any discrepancy the formula shall prevail. An asterisk is may be used in sub-formulas to indicate the bond which is connected to the core molecule as defined.

Unless specifically indicated, throughout the specification and the appended claims, a given chemical formula or name shall encompass tautomers and all stereo, optical and geometrical isomers (e.g. enantiomers, diastereomers, E/Z isomers etc...) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as for instance hydrates including solvates of the free compounds or solvates of a salt of the compound.

The term "C₁₋ₙ-alkyl", wherein n is an integer from 2 to n, either alone or in combination with another radical denotes an acyclic, saturated, branched or linear hydrocarbon radical with 1 to n C atoms. For example the term C₁₋₅-alkyl embraces the radicals H₃C-, H₃C-CH₂-, H₃C-CH₂-CH₂-, H₃C-CH(CH₃)-, H₃C-CH₂-CH₂-CH₂-, H₃C-CH₂-CH(CH₃)-, H₃C-CH(CH₃)-CH₂-, H₃C-C(CH₃)₂-, H₃C-CH₂-CH₂-CH₂-CH₂-, H₃C-CH₂-CH₂-CH(CH₃)-, H₃C-CH₂-CH(CH₃)-CH₂-, H₃C-CH(CH₃)-CH₂-CH₂-, H₃C-CH₂-C(CH₃)₂-, H₃C-C(CH₃)₂-CH₂-, H₃C-CH(CH₃)-CH(CH₃)- and H₃C-CH₂-CH(CH₂CH₃)-.

The term "C₁₋ₙ-haloalkyl", wherein n is an integer from 2 to n, either alone or in combination with another radical denotes an acyclic, saturated, branched or linear hydrocarbon radical with 1 to n C atoms wherein one or more hydrogen atoms are replaced by a halogene atom selected from among fluorine, chlorine or bromine, preferably fluorine and chlorine, particularly preferably fluorine. Examples include: CH₂F, CHF₂, CF₃.

The term "C₃₋ₙ-cycloalkyl", wherein n is an integer from 4 to n, either alone or in combination with another radical denotes a cyclic, saturated, unbranched hydrocarbon radical with 3 to n C atoms. For example the term C₃₋₇-cycloalkyl includes cyclopropyl, cyclobutyl, cyclopentyl, cyclohexyl and cycloheptyl.

### DETAILS OF THE INVENTION

**Dry Age-Related Macular Degeneration (dAMD)** is a progressive chronic disease affecting the central retina and a leading cause of vision loss in the elderly worldwide. Dry AMD is an advanced form of AMD is associated with the accumulation of drusen that lead to regions of geographic atrophy, that when involved the macula, cause devastating central vision loss. Although the exact mechanism is unknown, dry AMD patients frequently progress to wAMD through a process mediated by infiltration of macrophages into the retina that promote the release of pro-angiogenic factors and cause neovasculization.

**Wet Age-Related Macular Degeneration (wAMD)** is a form of advanced AMD that is characterized by neovascularization of the choroid that eventually tears the bruch's membrane, disrupts the retina, and causes vascular leakage and edema in the macular region thereby causing sudden central vision loss. Standard of care treatment targets VEGF-A, although approximately one-third still progress despite therapy. Moreover retinal toxicity has been demonstrated in conjunction with continuous or high dose anti VEGF therapy. Thus a treatment strategy with a more specific targeting of CNV is desirable. The expression of both CCR3 and its ligands have been specifically linked to the pathophysiology of this disease. Moreover CCR3 antagonism via NCE or NBE approaches has provided additional supportive evidence in pre-clinical studies for a role of CCR3 blockade as a potential therapeutic for this disease. Preclinical evidence suggests that compounds of fourmula **1** are fully efficacious in preventing laser induced neovascularization in pharmacology mouse models.

**Retinopathy of Prematurity (ROP)** is an eye disease that affects prematurely born babies who received intense neonatal care as a result of premature term birth. Both oxygen toxicity and local hypoxia are thought to contribute to the development of ROP. The underlying pathophysiology of the disease is that hypoxic conditions lead to stimulation of pro-angiogenic factors that cause disorganized growth of blood vessels with result in scarring and retinal detachment. Although ROP can be of mild intensity and fully recover without therapeutic intervention, it may lead to permanent blindness in serious cases. The exact cause of the disease is unknown but leading hypotheses are that supplemental oxygen causes either causes local retinal hypoxia through vasoconstriction which triggers neovascularization, or that normal vascular processes are blunted by supplemental oxygen, but when suddenly removed causes a rapid proliferation of vascular and fibrovascular disease. Current therapies include both surgical and therapeutic intervention to the disease in its severe form. Surgical therapy can include sclera buckling and/ or viterctomy for retinal detachment. Laser induced photocoagulation is however the mainstay of ROP treatment currently.

**Central Retinal Vein Occlusion (CRVO)** is a condition that occurs as a result of venous occusion preventing oxygen depleted blood from freely flowing out of the vasculature of the eye. Because limitation in flow of oxygen depleted blood, oxygen rich blood is inhibited from reaching the surface layers of the retina, and a hypoxic state ensures. The local hypoxia causes the surface layers of the retina to trigger proangiogenic factors. The release of these factors contributes to the the development of abnormal macular edema and neovascularization.

**Nasal Polyposis (NP)** is a chronic inflammatory disease of the upper respiratory tract characterized by an outgrowth of inflamed tissue into the nasal cavity, and although the exact etiology is unknown, it is known to have prevalence between 1 to 5% of adults (Settipane GA: Epidemiology of nasal polyps. Allergy Asthma Proc 1996, 17:231-236). NP typically presents in males 20 years of age or older and causes nasal obstruction, hyposmia, and recurrent infections with a significantly higher impact to quality of life than perennial allergic rhinitis (Li et al., Characterizing T-Cell Phenotypes in Nasal Polyposis in Chinese Patients, J Investig Allergol Clin Immunol 2009; Vol. 19(4): 276-282). Up to one third of all patients with NP are reported to have asthma however only 7% of asthma patients have NP. The predominate cell type implicated in NP is the eosinophil, although neutrophils are the predominate cell type found in NP in the far-east (Amar YG, Frenkiel S, Sobol SE: Outcome analysis of endoscopic sinus surgery for chronic sinusitis in patients having Samter's triad. J Otolaryngol 2000, 29:7-12). Samnter's triad (polyposis, asthma, and aspirin hypersensitivity) are known to comprise 10% of all NP, and are likely to be those with the highest recurrence rates (Naclerio et al., Medical and Surgical Management of nasal Polyps, Curr Opin Otolaryngol Head Neck Surg 2001, 9:27-36).

Treatment with locally acting nasal corticosteroids (nCS) is the current frontline treatment option, and has shown modest success. The reason for the lack of response with nasal steroids results from the underlying cause of the polyps that are non responsive to steroids (e.g. cystic fibrosis or ciliary dyskinesia), but perhaps additional limitation of the clinical utility of NP treatments is a high degree of nasal obstruction limiting intranasal distribution (Hellquist HB. Nasal polyps update. Histopathology. Allergy Asthma Proc. 1996;17:237-42). Long term treatment of NP with oral systemic corticosteroids (OCS) is efficacious, but is not widely adopted because of the known side effects. However OCS are often employed prior to surgery or initiation of treatment with intranasal steroids to shrink polyps for surgery or increase intranasal deliver of nCS. Patients who are non-responsive to medical management will require surgical management where nasal polyps are removed, and must continue chronic treatment with nasal steroids to avoid a recurrence of NP. A distinct subset of patients has a very high likelihood to recur, and those are patients with aspirin intolerance, fungal sinusitis, asthma, or cystic fibrosis. Because of the high impact on quality of life (e.g. complete loss olfactory function in severe NP), unimpressive response rates with nCS, undesirable side effect profile of OCS, and requirement for surgery for severe or non-responsive cases, the unmet medical need in NP is considered to be high. **Target Disease Link** - Histological evaluation reveals that nasal polyps can be divided into 4 types: edematous (eosinophilic), fibrotic non-eosinophilic), glandular, and atypical (Hellquist HB. Nasal polyps update. Histopathology. Allergy Asthma Proc. 1996;17:237-42). In the vast majority (80-90%) of histology evaluations in NP performed to date the etiology of NP has been characterized as having a strong eosinophillic component. Eosinophils initiate tissue damage by the release of cytotoxic substances like major basic protein, eosinophil cationic protein, and autocrine production of chemokines that perpetuate inflammatory processes. Not only the cytokines IL-1, IL-4, IL-5 and IL-8, but also and most importantly the chemokines eotaxin (CCL11) and RANTES (CCL5) have been ascribed a chemotactic potency for eosinophilia in the primary literature. Eotaxin and RANTES are known to signal on eosinophils through CCR3, and two additional eotaxins, namely eotaxin2 (CCL24) and eotaxin 3 (CCL26) have been shown to signal almost exclusively through the CCR3 receptor on eosinophils. Evaluation of eotaxin levels in nasal polyps has revealed a significant correlation between eotaxin levels and number of eosinophils in NP. The target disease link for a CCR3 antagonist is therefore provided in the primary literature, and data obtained in man with the compounds of formula **1** for the first time shows the ability to prevent eosinophil shape change in a dose and exposure dependant manner (internal data). The inhibition of eosinophil shape change represents a surrogate measurement of eosinophil activation and inhibition of eotaxin activity. It is therefore suggestive that a systemically available compound like the compounds of formula **1** will reduce eosinophil numbers in NP, reduce inflammation, and will be able to achieve symptomatic improve in NP where a high medical need has been identified.

**Eosinophilic Esophagitis (EoE)** is a chronic Th2 associated chronic inflammatory disease of the esophagus that currently affects at least 4 in 10,000 individuals (Noel RJ, Putnam PE, Rothenberg ME. Eosinophilic esophagitis. N Engl J Med. 2004;351:940-941). The diagnostic incidence has dramatically increased since 2000, paralleling an increase in endoscopy procedures (Prasad et al: Epidemiology of Eosinophilic Esophagitis over 3 Decades in Olmstead County, Minnesota. Clin Gastroenterol Hepatol. 2009, 7: 1055-1061). The hallmarks of the disease typically include dysphagia, food impaction, chest pain, and with unresolved heartburn despite high dose proton pump inhibitor therapy. Approximately one third of patients with EoE will require endoscopic removal of food impaction, and EoE pediatric studies have shown that the chronic nature of EoE manifests in behavioral changes in eating habits. The primary diagnosis is presentation with dysphagia associated with histological evaluation of endoscopic biopsies where >15 eosinophils are seen per high power field in the esophageal epithelium. Treatment for EoE usually involves several courses of high dose proton pump inhibitors since the initial misdiagnosis of GERD is common. Once EoE is confirmed via endoscopy in patients who are non-responsive to PPI's, treatment with the three D's is considered standard of care (Drugs, Diet, and esophageal dilation). The most commonly used drug is swallowed fluticasone iCS (440 ug bid), although there is currently no approved therapy for EoE. Histological differences between the proximal and distal esophagus indicate that sub-optimal deposition occurs with swallowed fluticasone. Although response rates are usually greater than 50%, high recurrence rates upon discontinuation of therapy indicates EoE is a chronic condition (Straumann A, Aceves SS, Blanchard C, Collins MH, Furuta GT, Hirano I, Schoepfer AM, Simon D, Simon H-U. Pediatric and adult eosinophilic esophagitis: similarities and differences. Allergy 2012; 67: 477-490). Trials with LTA's and anti TNF-a therapies have not yielded appreciable improvement in therapy (A. J. Lucendo et al., Montelukast Was Inefficient in Maintaining Steroid-Induced Remission in Adult Eosinophilic Esophagitis Dig Dis Sci (2011) 56:3551-3558). Mepolizumab (anti IL-5) therapy demonstrated a significant reduction in histological eosinophil numbers, but failed to demonstrate a significant symptomatic improvement in a small (n=11) exploratory study (Straumann A, Anti-interleukin-5 antibody treatment (mepolizumab) in active eosinophilic oesophagitis: a randomised, placebo-controlled, double-blind trial, Gut 2010;59:21-30). Although Mepolizumab lowered peripheral eosinophils by 5 fold, there was only a 2 fold reduction in biopsy eosinophils (e.g. chemotaxis leak), and patients had significantly higher peripheral eotaxin levels or auto-antibodies to anti IL-5 antibodies (A Straumann, Anti-interleukin-5 antibody treatment (mepolizumab) in active eosinophilic oesophagitis: a randomised, placebo-controlled, double-blind trial, Gut 2010;59:21-30).. These findings present hurdles for anti IL-5 therapy in EoE. EoE affects all ages with significant symptomatic and healthcare burden, representing a high unmet medical need. **Target Disease Link** - Eosinophils are not normally found in the esophagus epithelium, and the recognition of EoE as a Th2 type inflammatory disease was a large step forward towards understanding the disease (Straumann A, Aceves SS, Blanchard C, Collins MH, Furuta GT, Hirano I, Schoepfer AM, Simon D, Simon H-U. Pediatric and adult eosinophilic esophagitis: similarities and differences. Allergy 2012; 67: 477-490). Given the increased recognition of the disease and central role of biopsies in diagnosis of EoE, numerous studies have been summarized in the primary literature indicating that although eosinophils are the primary diagnostic marker. This pathophysiology is generally consistent for adult and pediatric EoE, with perhaps differences in the T-cell component of the disease. Although the hallmark Th2 cell types play some role, the eotaxin-3 axis (CCR3 and CCL26) has been most strongly implicated in being the key factors responsible for recruitment of eosinophils to the esophagus. The effector function of the eosinophils has been shown to be coupled with evidence for direct destruction of the esophagus epithelium. Degranulation of eosinophils causes the release of eosinophil cationic protein and eosinophil peroxidase, both of which have cytotoxic effects, and are implicated in the significant esophageal remodelling observed in EoE patients. Given the prominent role of the eosinophil in EoE, the signaling directly through the eotaxin-3 axis, and the clear role of CCR3 in eosinophil chemotaxis, the target disease link for EoE with a CCR3 antagonist is therefore provided in the primary literature. Data obtained in man with the compounds of formula **1** for the first time shows the ability to prevent eosinophil shape change in a dose and exposure dependant manner (internal data). The inhibition of eosinophil shape change represents a surrogate measurement of eosinophil activation and inhibition of eotaxin activity. It is therefore suggestive that a systemically available compound like the compounds of formula **1** will reduce eosinophil numbers in EoE, reduce inflammation, and will be able to achieve symptomatic improve in EoE where a high medical need has been identified.

Additionally compounds of formula 1 are expected to be useful in additional inflammatory diseases selected from eosinophillic gastroenteritis (e.g. eosinophilic gastritis and eosinophilic ententeritis), hypereosinophilic syndrome, and Churg Strauss syndrome as each of these diseases is associated with eosinophillic inflammation. Prevention of eosinophil chemotaxis into the affected tissues is predicted to resolve underlying inflammation and tissue damage.

### DOSAGES

A dosage range of the compound of formula **1** is usually between 100 and 1000 mg, in particular between 200 and 900 mg, 300 and 900 mg or 350 and 850 mg or 390 and 810 mg. It is possible to give one or two tablet, preferred are two tablets for a daily oral dosage of 100, 200, 300, 350, 400, 450, 500, 550, 600, 650, 700, 750, 800, 850, 900 mg, preferably 350, 400, 450, 750, 800, 850.

The dosages range can be achieved by one tablet or by two tablets; preferably two tablets are administered, each containing half of the dosage.

The application of the active ingredient may occur up to three times a day, preferably one or two times a day. Particular dosage strengths are 400 mg or 800 mg.

### EXAMPLES

Thus, present invention is directed to the use of compounds of formula 1 for the treatment of diseases selected from Nasal Polyposis and Eosinophilic Esophagitis (EoE). According to the rational above, this is connected with the ability of the compound to inhibit the CCR3 receptor. Ki values for the compounds of formula **1** (human Eotaxin-1 at human CCR3-Rezeptor) are shown in the table below.

As used herein, "activity" is intended to mean a compound demonstrating an inhibition of 50% at 1µM or higher in inhibition when measured in the aforementioned assays. Such a result is indicative of the intrinsic activity of the compounds as inhibitor of CCR3 receptor activity.

The examples of compounds of formula **1** can be synthesized according to the description of WO 2010 115836. The salts of these examples can be formed by crystallizing the free bases from a solution containing HCl. Preferably the examples 1, 2 3, 4, 5, 6, 7, 8, 9 and 10 are in form of the dihydrochloride.

| **Example #** | **Structure** | **hCCR3 Ki (nM)** | **2667** |
|---|---|---|---|
| 1. | 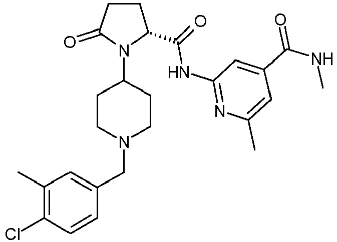 | 10,4 | 7 |
| 2. | 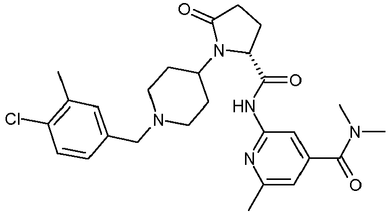 | 3,2 | 11 |
| 3. | 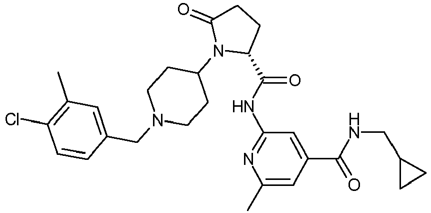 | 3,5 | 15 |
| 4. | 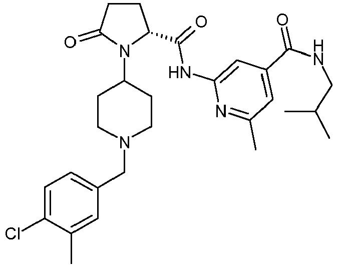 | 4,3 | 17 |
| 5. | 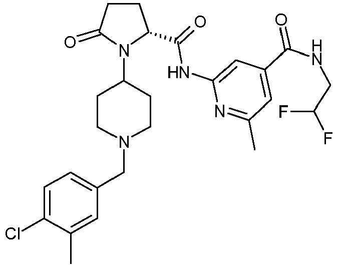 | 4,6 | 18 |
| 6. | 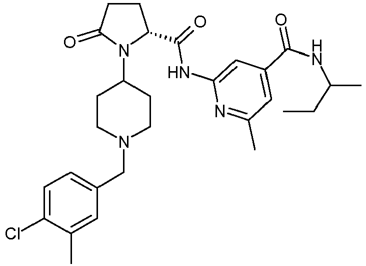 | 4,0 | 19 |
| 7. | 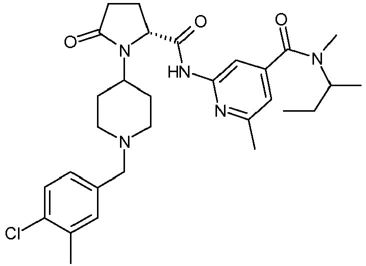 | 5,2 | 21 |
| 8. | 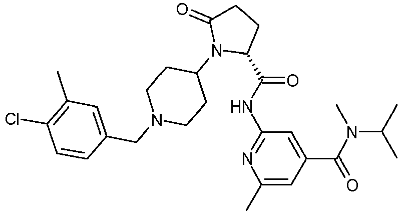 | 2,3 | 22 |
| 9. | 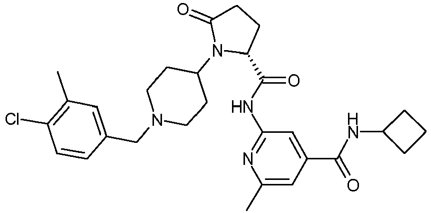 | 4,2 | 23 |
| 10. | 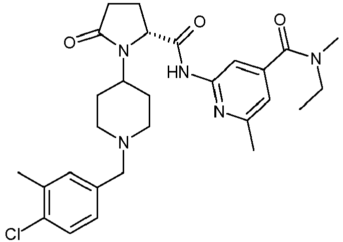 | 1,7 | 36 |

## Claims

1. Compound of formula **1** 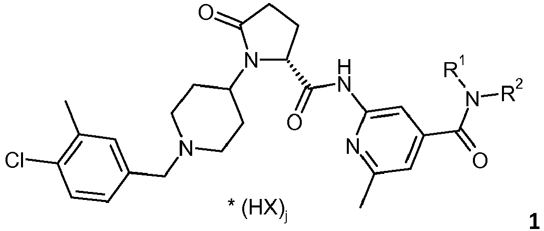 wherein
R¹ is H, C₁₋₆-alkyl, C₀₋₄-alkyl-C₃₋₆-cycloalkyl, C₁₋₆-haloalkyl;
R² is H, C₁₋₆-alkyl;
X is an anion selected from the group consisting of chloride or ½ dibenzoyltartrate
j is 1 or 2;
for use as a medicament for the treatment of diseases selected from nasal polyposis, eosinophilic esophagitis, eosinophillic gastroenteritis (e.g. eosinophilic gastritis and eosinophilic enteritis), hypereosinophilic syndrome and Churg Strauss syndrome.

2. Compound for use according to claim 1, wherein
R¹ is H, C₁₋₆-alkyl;
R² is H, C₁₋₆-alkyl;
X is an anion selected from the group consisting of chloride or ½ dibenzoyltartrate
j is 1 or 2.

3. Compound for use according to claims 1 or 2, wherein
R¹ is H, Methyl, Ethyl, Propyl, Butyl;
R² is H, Methyl, Ethyl, Propyl, Butyl;
X is an anion selected from the group consisting of chloride or ½ dibenzoyltartrate, preferably chloride;
j is 1 or 2, preferably 2.

4. Compound for use according to one of the claims 1 to 3, wherein
R¹ is H, Methyl, Ethyl, Propyl, Butyl;
R² is H, Methyl;
X is an anion selected from the group consisting of chloride or ½ dibenzoyltartrate, preferably chloride;
j is 1 or 2, preferably 2.

5. Compound for use according to one of the claims 1 to 4, wherein
R¹ is H, Methyl;
R² is H, Methyl;
X is an anion selected from the group consisting of chloride or ½ dibenzoyltartrate, preferably chloride;
j is 1 or 2, preferably 2.

6. Compound for use according to one of the claims 1 to 5, wherein X is chloride.

7. Compound for use according to one of the claims 1 to 6, wherein j is 2.

8. Compound of formula **1** according to any of claims 1 to 7 for use in the treatment of diseases selected from nasal polyposis, eosinophilic esophagitis, eosinophillic gastroenteritis (e.g. eosinophilic gastritis and eosinophilic enteritis), hypereosinophilic syndrome and Churg Strauss syndrome.

9. Compound for use according to claim 8 for the treatment of diseases selected from nasal polyposis and eosinophilic esophagitis.

## Patentansprüche

1. Verbindung gemäß Formel **1** 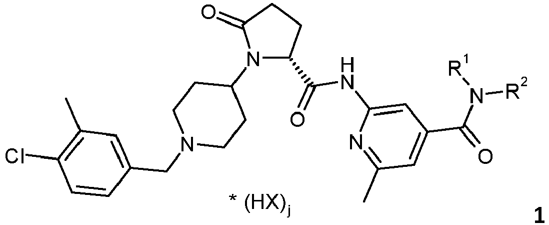 wobei
R¹ H, C₁₋₆-Alkyl, C₀₋₄-Alkyl-C₃₋₆-Cycloalkyl, C₁₋₆-Halogenalkyl ist;
R²H, C₁₋₆-Alkyl ist;
X ein Anion, ausgewählt aus der Gruppe bestehend aus Chlorid oder ½ Dibenzoyltartrat ist
j 1 oder 2 ist;
zur Verwendung als Medikament zur Behandlung von Krankheiten, ausgewählt aus Nasenpolyposis, eosinophiler Ösophagitis, eosinophiler Gastroenteritis (z.B. eosinophiler Gastritis und eosinophiler Enteritis), hypereosinophiles Syndrom und Churg-Strauss-Syndrom.

2. Verbindung zur Verwendung gemäß Anspruch 1, wobei die Verbindung
R¹ ist H, C₁₋₆-Alkyl;
R² ist H, C₁₋₆-Alkyl;
X ein Anion, ausgewählt aus der Gruppe bestehend aus Chlorid oder ½ Dibenzoyltartrat, ist;
j 1 oder 2 ist.

3. Verbindung zur Verwendung gemäß den Ansprüchen 1 oder 2, wobei
R¹ H, Methyl, Ethyl, Propyl, Butyl ist;
R² H, Methyl, Ethyl, Propyl, Butyl ist;
X ein Anion, ausgewählt aus der Gruppe, bestehend aus Chlorid oder ½ Dibenzoyltartrat, vorzugsweise Chlorid, ist;
j 1 oder 2, vorzugsweise 2 ist.

4. Verbindung zur Verwendung gemäß einem der Ansprüche 1 bis 3, wobei
R¹ H, Methyl, Ethyl, Propyl, Butyl ist;
R² H, Methyl ist;
X ein Anion, ausgewählt aus der Gruppe, bestehend aus Chlorid oder ½ Dibenzoyltartrat, vorzugsweise Chlorid, ist;
j 1 oder 2 vorzugsweise 2 ist.

5. Verbindung zur Verwendung gemäß einem der Ansprüche 1 bis 4, wobei
R¹ H, Methyl ist;
R² H, Methyl ist;
X ein Anion, ausgewählt aus der Gruppe, bestehend aus Chlorid oder ½ Dibenzoyltartrat, vorzugsweise Chlorid, ist;
j 1 oder 2 ist, vorzugsweise 2.

6. Verbindung zur Verwendung gemäß einem der Ansprüche 1 bis 5, wobei X Chlorid ist.

7. Verbindung zur Verwendung gemäß einem der Ansprüche 1 bis 6, wobei j 2 ist.

8. Verbindung der Formel 1 gemäß einem der Ansprüche 1 bis 7 zur Verwendung bei der Behandlung von Krankheiten, ausgewählt aus Nasenpolyposis, eosinophiler Ösophagitis, eosinophiler Gastroenteritis (z.B. eosinophiler Gastritis und eosinophiler Enteritis), hypereosinophiles Syndrom und Churg-Strauss-Syndrom.

9. Verbindung zur Verwendung gemäß Anspruch 8 zur Behandlung von Krankheiten, die aus nasaler Polyposis und eosinophiler Ösophagitis ausgewählt werden.

## Revendications

1. Composé de formule 1 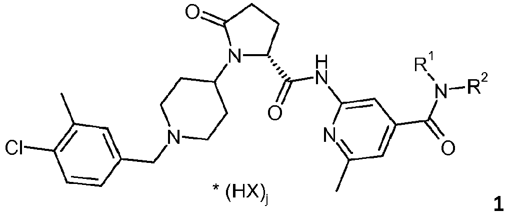 dans lequel
R¹ est H, un alkyle en C₁₋₆, un (alkyle en C₀₋₄)-(cycloalkyle en C₃₋₆), un halogénoalkyle en C₁₋₆ ;
R² est H, un alkyle en C₁₋₆ ;
X est un anion sélectionné dans le groupe consistant en le chlorure ou un hémitartrate de dibenzoyle ;
j est 1 ou 2 ;
pour son utilisation en tant que médicament pour le traitement de maladies sélectionnées parmi la polypose nasale, l'œsophagite éosinophile, la gastro-entérite éosinophile (par exemple, la gastrite éosinophile et l'entérite éosinophile), le syndrome hyperéosinophile et le syndrome de Churg Strauss.

2. Composé pour son utilisation selon la revendication 1, dans lequel
R¹ est H, un alkyle en C₁₋₆ ;
R² est H, un alkyle en C₁₋₆ ;
X est un anion sélectionné dans le groupe consistant en le chlorure ou un hémitartrate de dibenzoyle ;
j est 1 ou 2.

3. Composé pour son utilisation selon les revendications 1 ou 2, dans lequel
R¹ est H, un méthyle, un éthyle, un propyle, un butyle ;
R² est H, un méthyle, un éthyle, un propyle, un butyle ;
X est un anion sélectionné dans le groupe consistant en le chlorure ou un hémitartrate de dibenzoyle, de préférence le chlorure ;
j est 1 ou 2, de préférence 2.

4. Composé pour son utilisation selon l'une des revendications 1 à 3, dans lequel
R¹ est H, un méthyle, un éthyle, un propyle, un butyle ;
R² est H, un méthyle ;
X est un anion sélectionné dans le groupe consistant en le chlorure ou un hémitartrate de dibenzoyle, de préférence le chlorure ;
j est 1 ou 2, de préférence 2.

5. Composé pour son utilisation selon l'une des revendications 1 à 4, dans lequel
R¹ est H, un méthyle ;
R² est H, un méthyle ;
X est un anion sélectionné dans le groupe consistant en le chlorure ou un hémitartrate de dibenzoyle, de préférence le chlorure ;
j est 1 ou 2, de préférence 2.

6. Composé pour son utilisation selon l'une des revendications 1 à 5, dans lequel X est un chlorure.

7. Composé pour son utilisation selon l'une des revendications 1 à 6, dans lequel j est 2.

8. Composé de formule 1 selon l'une quelconque des revendications 1 à 7 pour son utilisation dans le traitement de maladies sélectionnées parmi la polypose nasale, l'œsophagite éosinophile, la gastro-entérite éosinophile (par exemple, la gastrite éosinophile et l'entérite éosinophile), le syndrome hyperéosinophile et le syndrome de Churg Strauss.

9. Composé pour son utilisation selon la revendication 8 pour le traitement de maladies sélectionnées parmi la polypose nasale et l'œsophagite éosinophile.
